# EUROPEAN PATENT APPLICATION

(11) **EP 3 040 053 A1**
(43) Date of publication of application: **06.07.2016**
(21) Application number: 14841103.6
(22) Date of filing: 22.08.2014
(51) Int. Cl.: A61F 2/24

(54) **BASE MATERIAL FOR FORMING CONNECTIVE TISSUE STRUCTURE AND METHOD FOR PRODUCING CONNECTIVE TISSUE STRUCTURE**

(30) Priority: 30.08.2013 JP 2013178919
(71) Applicant: National Cerebral and Cardiovascular Center, Suita-shi, Osaka 565-8565 (JP)
(72) Inventor: NAKAYAMA, Yasuhide, Suita-shi Osaka 565-8565 (JP); OIE, Tomonori, Suita-shi Osaka 565-8565 (JP)
(74) Representative: Müller Hoffmann & Partner
(86) International application number: PCT/JP2014/071985
(87) International publication number: WO 2015/029891

(57) **Abstract**

The present invention provides a base material for forming a connective tissue structure which can accurately cut the connective tissue structure to a predetermined shape and a method for producing the connective tissue structure. A cutting-line forming groove (28) is formed in a surface of a base material (19). The base material (19) is placed under an environment in which a biological tissue material is present. A membranous connective tissue structure (20) is formed on the surface of the base material (19). At that time, a connective tissue (30) is made to invade into the cutting-line forming groove (28). A cutting line (31) for cutting is formed in the connective tissue structure (20). The connective tissue structure (20) is accurately cut along the cutting line (31) to a predetermined shape.

## Description

### Technical Field

The present invention relates to a base material for forming a connective tissue structure for forming a membranous connective tissue structure and a method for producing a connective tissue structure.

### Background Art

Many studies of regenerative medicine for regenerating cells, tissues and organs lost by diseases and accidents by artificial materials or cells have been made. Usually, a body has a self-defense function, and if a foreign substance such as a thorn invades to a shallow position in the body, the body tries to push it out, but if the foreign substance invades to a deep position in the body, it is known that fibroblasts gather around the foreign substance and form a capsule of a connective tissue structure made mainly of the fibroblasts and collagen and cover the foreign substance so as to isolate it in the body. Several methods for forming a tissue derived from a living body using living cells in a living body by using the self-defense reaction of the latter as above are reported (see Patent Literatures 1 to 3).

Moreover, Patent Literature 4 discloses a stent in which a valve body is formed integrally with a connective tissue structure layer covering a stent intermediate body by covering the stent intermediate body with a connective tissue structure layer and by providing a notch in the tissue structure.

### Citation List

### Patent Literature

Patent Literature 1: Japanese Patent Laid-Open No. 2007-312821
Patent Literature 2: Japanese Patent Laid-Open No. 2008-237896
Patent Literature 3: Japanese Patent Laid-Open No. 2010-094476
Patent Literature 4: Japanese Patent Laid-Open No. 2007-037763 (paragraphs 0037, 0044, 0045 and Figure 1)

### Summary of Invention

### Technical Problem

The invention described in Patent Literature 4 is for forming a valve body by applying cutting to an artificially formed connective tissue structure, and since the connective tissue structure is flexible and easy to be deformed, accurate cutting of the connective tissue structure to a predetermined shape tends to be a work which is relatively difficult and requires care for that portion. Moreover, since a lot of time and labor are required for artificially forming a connective tissue structure, a method for cutting the connective tissue structure to an accurate shape without a failure is in demand.

The present invention has an object to provide a base material for forming a connective tissue structure which can accurately cut the connective tissue structure to a predetermined shape and a method for producing a connective tissue structure.

### Solution to Problem

In order to achieve the aforementioned object, a base material for forming a connective tissue structure according to the present invention is for forming a membranous connective tissue structure on a surface of the base material by being placed under an environment in which a biological tissue material is present, and a working mark forming portion for forming a mark when the connective tissue structure is worked is formed.

According to the aforementioned constitution, since the working mark forming portion is formed on the surface of the base material, when the membranous connective tissue structure is to be formed on the base material surface, the mark can be formed by causing a connective tissue to invade into the working mark forming portion, and the membranous connective tissue structure which can be accurately worked to the predetermined shape at the position of the mark can be formed. The working mark forming portion may be any one of a recess, a projection or a combination of them but by forming the working mark forming portion on the base material surface by a recess, the mark can be constituted by a projection made of the connective tissue, and the mark can be made more distinctive than constitution of the mark by the recess, whereby working of the membranous connective tissue structure can be made easier.

The term "biological tissue materials" here means substances required for forming a desired tissue derived from a living body and includes animal cells such as fibroblasts, smooth muscle cells, endothelial cells, stem cells, ES cells, iPS cells and the like, various proteins (collagen, elastin), sugars such as hyaluronic acid and other various physiologically active substances present in a living body such as cell growth factors, cytokine and the like. The "biological tissue materials" include those derived from mammals such as humans, dogs, cows, pigs, goats, sheep and the like, birds, fish and other animals or artificial material equal to them.

Moreover, the phrase "under the environment in which a biological tissue material is present" means an artificial environment containing the biological tissue material in a living body (under the skin of a limb part, a shoulder part, a back part or a belly part or implantation in an abdominal cavity, for example) of an animal (mammals such as humans, dogs, cows, pigs, goats, sheep and the like, birds, fish and other animals) or outside of a living body of an animal.

Moreover, the term "connective tissue" usually means a tissue having collagen as a main component and refers to a tissue formed in a living body but in the description in this Description and claims, it is a concept including a tissue when a tissue corresponding to a connective tissue formed in a living body is formed under the environment outside of the living body.

As the working mark forming portion, a cutting-line forming groove for forming a cutting line when the connective tissue structure is to be cut can be exemplified.

According to this constitution, since the cutting-line forming groove is formed on the base material surface, when the membranous connective tissue structure is to be formed on the base material surface, the connective tissue can be made to invade into the cutting-line forming groove so as to form a cutting line, and the membranous connective tissue structure in which a predetermined shape can be accurately cut along the cutting line can be formed.

Moreover, since the connective tissue is made to invade into the cutting-line forming groove on the base material surface so as to form the cutting line, the cutting line can be constituted by a projection made of the connective tissue, and the cutting line can be made more distinctive than a case in which the cutting line is constituted having a groove shape, and cutting of the membranous connective tissue structure can be made easier. Moreover, if the connective tissue structure is cut along an outer side of this cutting line so that the projection cutting line is left, a cutting edge of the membranous connective tissue structure can be reinforced by the projection constituting the cutting line.

Moreover, as the working mark forming portion, a folding-back mark forming portion for forming a folding-back mark when the connective tissue structure is folded can be exemplified.

According to this constitution, since the folding-back mark forming portion is formed on the base material surface, when the membranous connective tissue structure is formed on the base material surface, the connective tissue can be made to invade into the folding-back mark forming portion so as to form a folding-back mark, and the membranous connective tissue structure which can accurately perform folding-back to a predetermined shape at the position of the folding-back mark can be formed.

Moreover, as the working mark forming portion, a fixing mark forming portion for forming a fixing mark when the connective tissue structure is fixed can be exemplified.

According to this constitution, since the fixing mark forming portion is formed on the base material surface, when the membranous connective tissue structure is formed on the base material surface, the connective tissue can be made to invade into the fixing mark forming portion so as to form the fixing mark, and the membranous connective tissue structure which can accurately perform fixing to a predetermined shape at the position of the fixing mark can be formed.

Furthermore, a projection forming groove forming a projection on the connective tissue structure may be formed on the base material surface separately from the working mark forming portion.

According to this constitution, since the projection forming groove is formed separately from the working mark forming portion, in addition to the working mark, the projection can be formed on the connective tissue structure separately from that, and this another projection can be made another mark for grasping the shape and direction of the connective tissue structure and finding the working mark more easily.

Furthermore, when the cutting-line forming groove is to be formed as the working mark forming portion, a projection forming groove for forming a projection in the connective tissue structure may be formed on the base material surface separately from the cutting-line forming groove, and the projection forming groove may be provided in parallel with the cutting-line forming groove in a groove width direction.

According to this constitution, another projection adjacent to the projection constituting the cutting line in the groove width direction can be formed in the connective tissue structure formed on the base material surface, and since a plurality of projections including the cutting line are formed, the cutting line of the connective tissue structure can be found more easily. Moreover, by cutting the connective tissue structure along the cutting line, another projection can be left along the cutting line, and this left projection can reinforce the cutting edge of the connective tissue structure. Moreover, instead of the cutting line, by cutting the connective tissue structure along another projection, the connective tissue structure can be formed having another size or shape.

Furthermore, the projection forming groove may be formed as a thickness-increasing groove which increases thickness and reinforces the connective tissue structure. According to this constitution, by forming the projection by the projection forming groove as the thickness-increasing groove, the connective tissue structure can be increased in thickness and reinforced, and the projection having a function of grasping the shape and direction of the connective tissue structure more easily and a function of increasing thickness and reinforcing the connective tissue structure can be formed.

Moreover, the present invention provides a method for producing the membranous connective tissue structure made of the connective tissue. Specifically, the method includes an installing process of placing a base material having a cutting-line forming groove on the base material surface under an environment in which a biological tissue material is present, a forming process of forming a cutting line by causing this connective tissue to invade into the cutting-line forming groove while the connective tissue is formed around the base material, a taking-out process of taking out the base material covered with the connective tissue from the environment, a separating process of separating and taking out the connective tissue covering the base material surface as the membranous connective tissue structure, and a cutting process of cutting the connective tissue structure along the cutting line.

According to the aforementioned constitution, in the forming process, the connective tissue can be made to invade into the cutting-line forming groove in the base material surface so as to form the cutting line at an accurate position of the membranous connective tissue structure formed on the base material surface, and in the cutting process, the connective tissue structure can be cut along the cutting line, whereby the membranous connective tissue structure can be accurately cut to a predetermined shape.

Moreover, as a method for producing the membranous connective tissue structure, not only that the cutting line is formed, but also other working marks such as the folding-back mark and the fixing mark may be formed.

That is, the present invention is a method for producing the membranous connective tissue structure made of the connective tissue and provides a method for producing a connective tissue structure, including an installing process of placing a base material having a working mark forming portion on the base material surface under an environment in which a biological tissue material is present, a forming process of forming a mark by causing the connective tissue to invade into the working mark forming portion while the connective tissue is formed around the base material, a taking-out process of taking out the base material covered with the connective tissue from the environment, a separating process of separating and taking out the connective tissue covering the base material surface as the membranous connective tissue structure, and a working process of working the connective tissue structure at the position of the mark.

### Advantageous Effects of Invention

As described above, according to the present invention, since the cutting-line forming groove is formed in the base material surface forming the membranous connective tissue structure so as to form the cutting line in the connective tissue structure, the connective tissue structure can be cut along this cutting line. As a result, the membranous connective tissue structure requiring a lot of time and labor to be artificially formed and which is flexible and easily deformable can be accurately cut to a predetermined shape without a failure.

### Brief Description of Drawings

[Figure 1] Figure 1 is a perspective view of an artificial valve formed by using a base material for forming a connective tissue structure according to the present invention when seen from a tip end side.
[Figure 2] Figure 2 is a perspective view of the artificial valve when seen from a base end side.
[Figure 3] Figure 3 is a longitudinal sectional view illustrating attachment of the artificial valve to a heart.
[Figure 4] Figure 4 is a perspective view illustrating a closed valve state of the artificial valve.
[Figure 5] Figure 5 is a perspective view of the base material for forming the connective tissue structure according to the present invention.
[Figure 6] Figure 6 is a view illustrating procedures for producing the artificial valve.
[Figure 7] Figure 7 is a perspective view of the base material for forming the connective tissue structure of another embodiment.
[Figure 8] Figure 8 is a perspective view of the base material for forming the connective tissue structure of still another embodiment.
[Figure 9] Figure 9 is a perspective view of an artificial valve having a folding structure.
[Figure 10] Figure 10 is a perspective view of the base material for forming the connective tissue structure used for production of the artificial valve having the folding structure.
[Figure 11] Figure 11 is a view illustrating a procedure of producing the artificial valve having the folding structure.

### Description of Embodiments

First to fourth embodiments of a base material for forming a connective tissue structure according to the present invention and a method for producing a connective tissue structure will be described below by using the attached drawings.

### [First Embodiment]

First, an artificial valve as a membranous connective tissue structure produced by using a base material for forming a connective tissue structure and a method for production of this embodiment will be described.

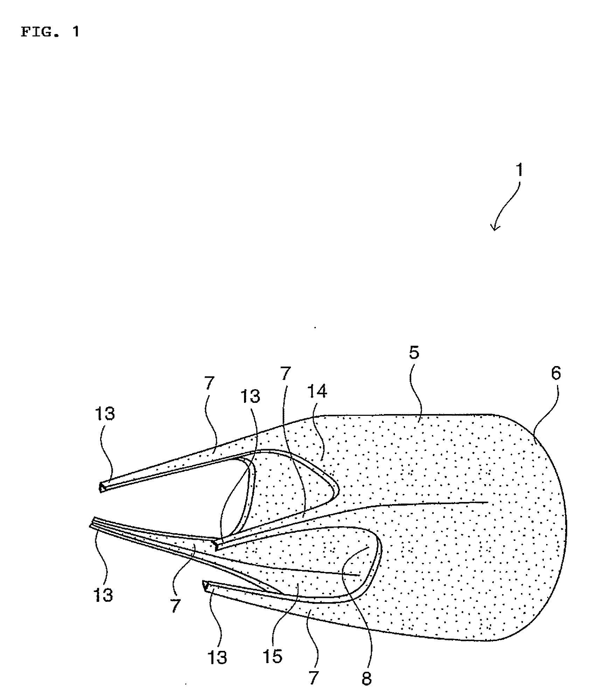

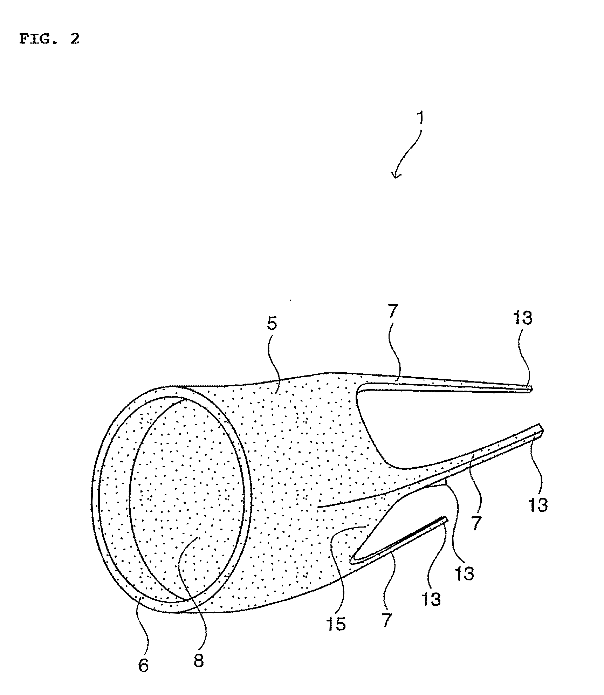

As illustrated in Figures 1 to 4, the artificial valve 1 is implantable at a portion with a small length in a flow direction and is to be used as a mitral valve partitioning a left ventricle 3 from a left atrium 4 of a heart 2 and includes a cylindrical valve body 5 made of a connective tissue, and a base end portion of the valve body 5 is made a fixing portion 6 capable of fixing the artificial valve 1 to an implantation site, and string-shaped tendinous chords 7 are extended from a plurality of spots in a circumferential direction of a tip end portion of the valve body 5.

The valve body 5 has a cylindrical shape of a substantially truncated cone whose circumferential length on the tip end side is set smaller than that on the base end side and is constituted such that a center hole 8 is pushed open and the valve is opened, while the center hole 8 is pushed and crushed and the valve is closed by a pressure of a fluid passing through the center hole 8 thereof.

The fixing portion 6 is increased in thickness more than the other portions in the valve body 5 so as to be reinforced and has sufficient strength to be fixed to the heart 2 or the like without losing flexibility of the valve body 5.

The tendinous chord 7 has a string-like smooth tapered shape, and four tendinous chords 7 are extended to the tip end side one by one from four spots set at substantially equal intervals in the tip end of the valve body 5, and it is constituted such that, when the artificial valve 1 is to be implanted, its tendinous-chord tip end portion 13 is fixed to the tip end side rather than the valve body 5. The tip end of the valve body 5 is retained at the implantation site through the tendinous chords 7, and moreover, by bringing the fixation positions of the four tendinous chords 7 closer vertically and by setting them separately from each other to right and left, the valve body 5 is made crushable vertically. The valve body 5 crushed vertically is constituted such that its upper half is made an anterior cusp 14 and a lower half is made a posterior cusp 15, and that the anterior cusp 14 and the posterior cusp 15 are brought into contact and the valve is closed.

The tendinous chord 7 is constituted by the same "connective tissue" as the "connective tissue" constituting the valve body 5, but in the description of this Description, the term "tendinous chord" is used as it is.

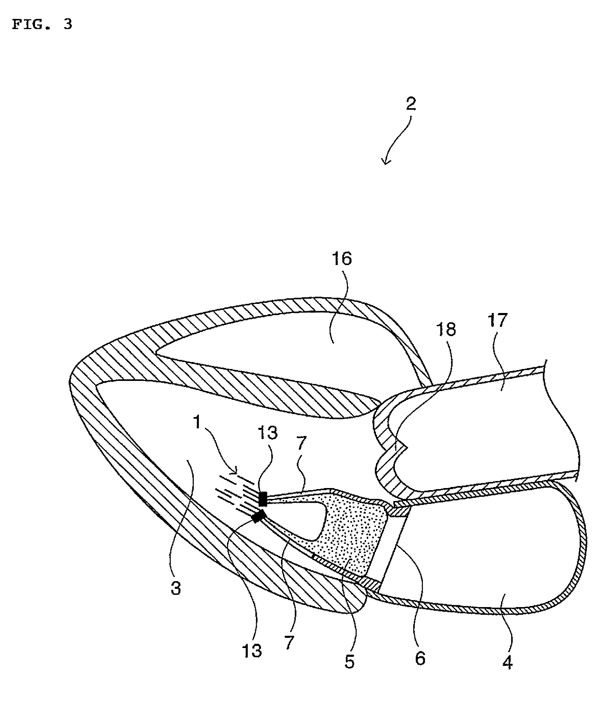

The artificial valve 1 is implanted as an artificial mitral valve to the heart 2 by fixing the fixing portion 6 of the valve body 5 between the left ventricle 3 and the left atrium 4 of the heart 2 and by fixing the tendinous-chord tip end portion 13 of the tendinous chord 7 to a ventricle wall of the left ventricle 3 (see Figure 3). Here, Figure 3 is a sectional view of the heart 2 and illustrates a section on a vertical plane passing the left ventricle 3, the left atrium 4, a right ventricle 16, an aorta 17, and an aortic valve 18.

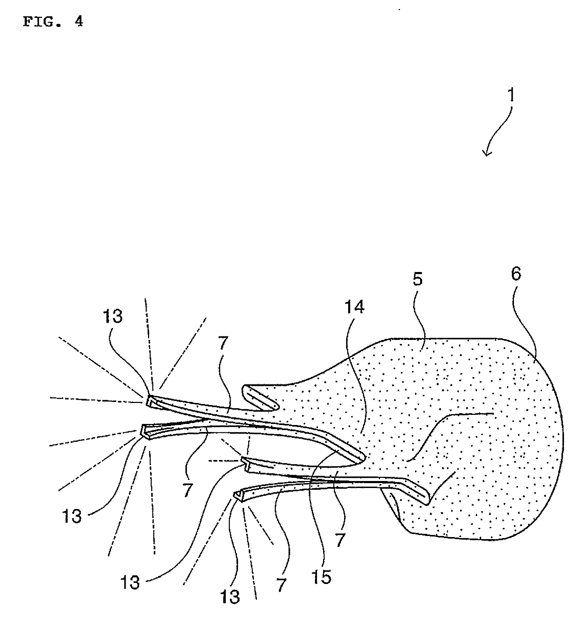

The artificial valve 1 implanted in the heart 2 as the artificial mitral valve has the center hole 8 crushed by a pressure of blood applied to an outer surface of the substantially truncated-cone cylindrical valve body 5 when the left ventricle 3 contracts, the anterior cusp 14 and the posterior cusp 15 are brought into contact and the valve is closed, and a blood flow flowing from the left ventricle 3 toward the left atrium 4 is inhibited (see Figure 4). On the other hand, when the blood flow is to flow from the left atrium 4 to the left ventricle 3, the center hole 8 of the valve body 5 is pushed open by the blood flow, and the valve is opened.

In this artificial valve 1, since the tip end portion of the valve body 5 is retained by the tendinous chords 7, the tip end portion of the valve body 5 is prevented from being pushed into the left atrium 4 by the pressure of the blood flow when the valve is closed. Moreover, since the fixing positions of the upper left and lower left tendinous chords 7 are brought closer and the fixing positions of the upper right and lower right tendinous chords 7 are brought closer, the tendinous chords 7 are prevented from interfering closing of the valve body 5.

Subsequently, a base material 19 for forming a connective tissue structure according to the present invention used when the membranous connective tissue structure as the aforementioned artificial valve 1 is produced will be described.

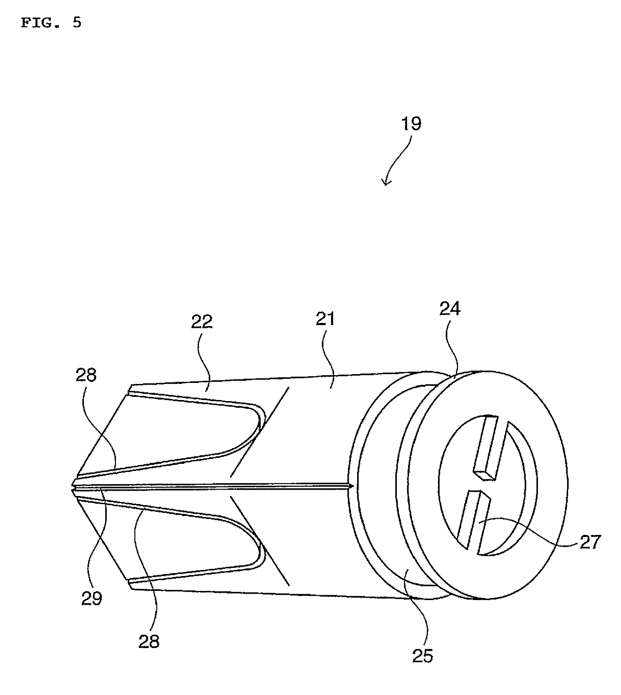

As illustrated in Figure 5, the base material 19 is for forming the membranous connective tissue structure 20 which can be worked to the artificial valve 1 on the surface thereof by placing it under an environment in which a biological tissue material is present, and on the base material surface, a cutting-line forming groove 28 for forming a projection as a cutting line 31 when the connective tissue structure 20 is cut and a thickness-increasing groove 29 for increasing the thickness for reinforcement by forming a projection for reinforcement in the connective tissue structure 20 are formed separately.

This base material 19 includes a valve-body forming portion 21 for forming a portion which can be worked to the cylindrical valve body 5 in the connective tissue structure 20 and a tendinous-chord forming portion 22 for forming a portion which can be worked to the string-shaped tendinous chord 7 extended from the tip end portion of the valve body 5 in the connective tissue structure 20 and is constituted having a columnar shape in general by causing the valve-body forming portion 21 and the tendinous-chord forming portion 22 to be adjacent to each other in a direction of a center axis.

The valve-body forming portion 21 has its base-end section formed having a circular shape and a tip-end section formed having a square shape having a diagonal line with the substantially same length as a diameter of the base-end section and has a shape in which the both sections are smoothly continued and a circumferential length is set smaller on the tip end side than on the base end side.

On the base end portion of the valve-body forming portion 21, a notch 23 continuing in a circumferential direction is formed, and the base end side of the notch 23 is closed by a flange 24 and a groove portion 25 is constituted. The flange 24 has a disc shape having the substantially same diameter as the base end of the valve-body forming portion 21, and a circular projection 26 projecting from its one surface is constituted capable of being fitted in a fitting hole open on the base end side of the valve-body forming portion 21 and of being detachably attached to the base end side of the valve-body forming portion 21. In Figure 5, reference numeral 27 denotes a knob for detachably attaching the flange 24.

The tendinous-chord forming portion 22 is a cuboid having the same sectional shape and the same size as the tip end of the valve-body forming portion 21 and is formed adjacent to the tip end side of the valve-body forming portion 21. On each side surface of the tendinous-chord forming portion 22, on the connective tissue structure 20 formed on the surface, the cutting-line forming groove 28 for forming a projection as the cutting line 31 when this is cut to the tendinous chord 7 is formed. This cutting-line forming groove 28 is formed having a substantially arch form so as to partition a tapered range in the vicinity of a corner of the tendinous-chord forming portion 22 from the center portion.

On each corner portion of the valve-body forming portion 21 and the tendinous-chord forming portion 22, the thickness-increasing groove 29 for increasing the thickness for reinforcement by forming a projection at the corner portions of the valve body 5 and the tendinous chords 7 formed on the surface of the base material 19 is formed continuously in a center axis direction. By increasing the thickness and reinforcing the corner portions of the valve body 5 and the tendinous chords 7 by this thickness-increasing groove 29, the corner portions that can be weak-point portions in the valve body 5 and the tendinous chords 7 are reinforced. Moreover, regarding the connective tissue structure 20 before cutting, the positions of the corner portions are indicted by the projections, and the direction of the connective tissue structure 20 and the position of the cutting line can be grasped easily.

For a material of the base material 19, a resin having strength (hardness) which is not largely deformed when being implanted in a living body and which is chemically stable and resistant to a load such as disinfection and has no or little effluent which stimulates a living body is preferable, and it includes a silicone resin, an acrylic resin or the like, for example, but are not limited to that. Since a thickness of the artificial valve 1 is determined by an outer diameter of the base material 19, the diameter can be changed in accordance with an intended thickness.

Subsequently, the method for producing the artificial valve 1 as the membranous connective tissue structure will be described by using the aforementioned base material 19 for forming a connective tissue structure.

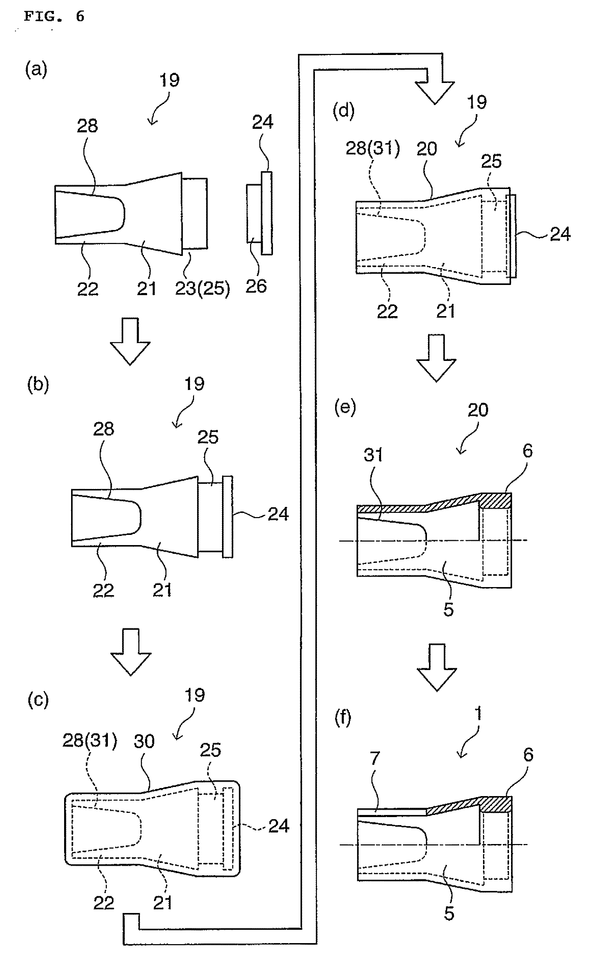

As illustrated in Figure 6, this production method includes an "assembling process" of assembling the columnar base material 19 while the groove portion 25 is being constituted, an "installing process" of placing the base material 19 having the cutting-line forming groove 28 in the base material surface under the environment in which the biological tissue material is present, a "forking process" of forming the cutting line 31 by causing the connective tissue 30 to invade into the cutting-line forming groove 28 while the connective tissue 30 is formed around the base material 19, a "taking-out process" of taking out the base material 19 covered with the connective tissue 30 from the environment, a "separating process" of separating and taking out the cylindrical connective tissue structure 20 from the base material 19, and a "cutting process" of cutting a portion formed on the surface of the tendinous-chord forming portion 22 in the connective tissue structure 20 along the cutting line 31 and extending the string-shaped tendinous chords 7 from the cylindrical valve body 5 formed on the surface of the valve-body forming portion 21.

In Figure 6, (a) to (d) illustrate side views, and (e) and (f) illustrate sectional views in upper halves and side views in lower halves.

### <Assembling process>

The circular projection 26 is fitted in the fitting hole on the base end side of the valve-body forming portion 21 of the base material 19, the flange 24 is mounted on the base end side of the valve-body forming portion 21, and the base material 19 is assembled. As a result, the base end side of the notch 23 is closed by the flange 24, and the groove portion 25 is constituted (Figures 6(a) and 6(b)).

### <Installing process>

The base material 19 having the cutting-line forming groove 28 on the base material surface is placed under the environment in which the biological tissue material is present (Figure 6(b)). The environment in which the biological tissue material is present is in a living body of an animal (implantation under the skin or into an abdominal cavity, for example) or under an artificial environment such as in a solution in which a biological tissue material floats outside the living body of an animal. As the biological tissue material, those derived from mammals such as humans, dogs, cows, pigs, goats, rabbits, sheep and the like, birds, fish and other animals or artificial material can be also used.

When the base material 19 is to be implanted in an animal, it is performed in a minimum incision under sufficient anesthesia, and a wound is sewn after the implantation. As an insertion portion of the base material 19, inside an abdominal cavity having a volume for containing the base material 19 or under the skin of a limb part, a shoulder part, a back part or a belly part is preferable. Moreover, it is preferable that the implantation is performed with a low-invasive method and in a minimum incision under sufficient anesthesia in view of the spirit of protection of animals.

Moreover, when the base material 19 is to be placed under the environment in which the biological tissue material is present, it is only necessary to perform cell culture in accordance with a known method under a clean environment with various culture conditions ready.

### <Forming process>

After the installing process, as predetermined time elapses, the membranous connective tissue 30 is formed around the base material 19 (Figure 6(c)). The connective tissue 30 invades into the groove portion 25 and forms the fixing portion 6 having increased thickness.

Moreover, as the connective tissue 30 invades into the cutting-line forming groove 28, a projection is formed on the connective tissue structure 20 and the cutting line 31 is constituted, and as the connective tissue 30 invades into the thickness-increasing groove 29, the projection is formed at the corner portion of the connective tissue structure 20 and the thickness is increased and reinforced. The connective tissue 30 is constituted by a fibroblast and an extracellular matrix such as collagen.

### <Taking-out process>

After the connective tissue 30 is sufficiently formed after the forming process of the predetermined time has elapsed, the taking-out process of taking out the base material 19 from the environment in which the biological tissue material is present is performed. The base material 19 taken out from the environment in which the biological tissue material is present is covered with a membrane made of the connective tissue 30 on the whole surface including its both end surfaces.

### <Separating process>

The connective tissue 30 on the surface of the both end portions of the base material 19 is removed (Figure 6(d)), and after that, the flange 24 is removed from the base end side of the valve-body forming portion 21, the base end side of the groove portion 25 with which the fixing portion 6 is still fitted is opened, and the remaining connective tissue 30 covering the circumferential surface of the base material 19 is separated and taken out as the cylindrical connective tissue structure 20 from the surface of the base material 19 (Figure 6(e)).

### <Cutting process>

By using the projection formed by the thickness-increasing groove 29 as a mark, the cutting line 31 is found while the position of the corner portion of the connective tissue structure 20 is grasped, and the connective tissue structure 20 is cut along this cutting line 31 so as to form the string-shaped tendinous chords 7, whereby the artificial valve 1 in which the tendinous chords 7 are extended from the valve body 5 is obtained (Figure 6(f)). In this cutting process, if the cutting is performed so that the projection constituting the cutting line 31 is left, the effect of reinforcing the cutting edge by the left projection can be expected.

When the produced artificial valve 1 is subjected to heterograft, in order to prevent rejection after the implantation, immunogen removing treatments such as decellularization treatment, dehydration treatment, fixation treatment and the like are preferably applied. The decellularization treatment includes methods of cleaning by causing the extracellular matrix to liquate by ultrasonic treatment, surfactant treatment, enzyme treatment such as collagenase and the like, the dehydration treatment includes methods of cleaning with water-soluble organic solvent such as methanol, ethanol, isopropyl alcohol and the like, and the fixation treatment method includes a method of treatment using aldehyde compound such as glutaraldehyde and formaldehyde.

### [Second Embodiment]

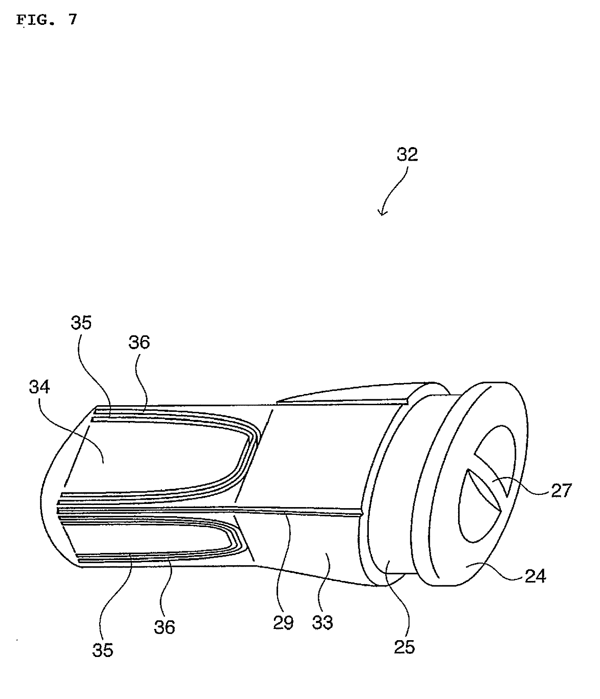

This embodiment has substantially the same constitution as the first embodiment, but as illustrated in Figure 7, as the base material for forming a connective tissue structure, a flat-shaped base material 32 is employed instead of the base material 19 having a circular base end section and a square tip end section.

A valve-body forming portion 33 of the base material 32 has its base end section formed having an elliptic shape and its tip end section formed having a rectangular shape with a size inscribing the ellipse of the base end section so as to form a shape in which the both sections are smoothly continued, and the circumferential length on the tip end side is set smaller than on the base end side. Moreover, a tendinous-chord forming portion 34 of the base material 32 has a cuboid section with the same shape and the same size as those of the tip end of the valve-body forming portion 33.

In a side surface of the base material 32, double grooves are formed, and the groove on an inner side of the arch shape is made a cutting-line forming groove 35, while the groove on an outer side of the arch shape is made a thickness-increasing groove 36 for increasing the thickness and reinforcing a side edge of the tendinous chord 7. As a result, the cutting-line forming groove 35 and the thickness-increasing groove 36 are provided in parallel in a groove width direction, and double projections including the cutting line 31 are formed in the connective tissue structure 20 so that the cutting line 31 can be made distinctive and found easily when the connective tissue structure 20 is to be cut.

In the artificial valve 1 formed by using the base material 32, the projection formed by the thickness-increasing groove 36 is located along a cutting edge and reinforces a side edge of the tendinous chord 7. Moreover, by using a thickness-increasing line formed by the thickness-increasing groove 36 on the outer side of the arch shape as a cutting line, a tendinous chord having a width smaller than the tendinous chord 7 formed by cutting the connective tissue structure 20 along the cutting line formed by the cutting-line forming groove 35 can be formed.

Moreover, in the artificial valve 1, since its tendinous-chord forming portion 34 has a rectangular section, the tendinous chords 7 can be arranged at unequal intervals so as to bring base ends of the upper and lower tendinous chords 7 in the valve open state closer to each other, and resistance by the tendinous chord 7 when the valve body 5 is vertically crushed and closed can be made smaller.

### [Third Embodiment]

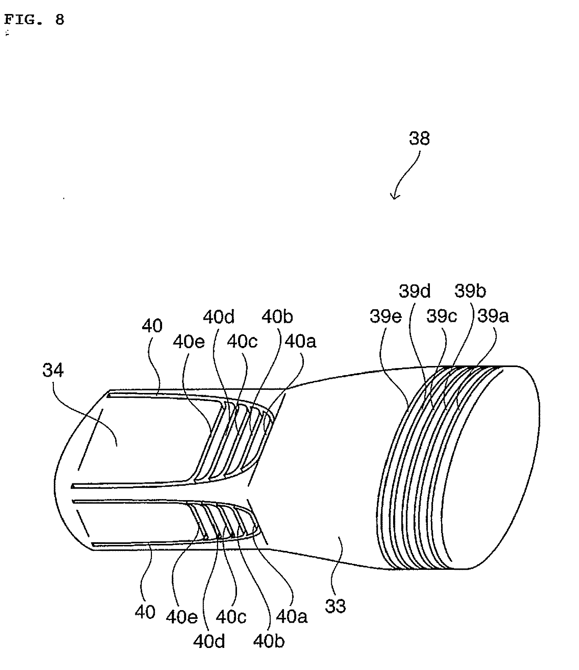

This embodiment has substantially the same constitution as the second embodiment, but the thickness of the fixing portion of the artificial valve is not increased but it is formed with a thickness to the same degree of the other portions. In this embodiment, as illustrated in Figure 8, instead of the base materials 19 and 32, the artificial valve is formed by using a base material 38 not having the groove portion 25 in the base end portion of the valve-body forming portion 33.

In the base end portion of the valve-body forming portion 33 of the base material 38, a plurality of cutting-line forming grooves 39a, 39b, 39c, 39d, and 39e for a valve body continuing in the circumferential direction are formed so as to form a plurality of cutting lines in the connective tissue structure 20. In the plurality of the cutting lines, by cutting the connective tissue structure 20 along the cutting line selected as appropriate in accordance with an implantation site, a size of the valve body 5 can be adjusted. Moreover, by cutting the valve body 5 diagonally using the plurality of cutting lines as a mark, an opening shape of the fixing portion of the valve body 5 can be also adjusted.

In the tendinous-chord forming portion 34 of the base material 38, a cutting-line forming groove 40 having an arch shape is formed, and the vicinity of the top part of the arch shape is formed by branching into a plurality of branch grooves 40a, 40b, 40c, 40d, and 40e so as to form the cutting lines branching in plural in the connective tissue structure 20. In the branching cutting lines, by cutting the connective tissue structure 20 along the cutting line selected as appropriate in accordance with the implantation site, the size of the valve body 5 and the length of the tendinous chord 7 can be adjusted.

### [Fourth Embodiment]

This embodiment has substantially the same constitution as the first to third embodiments, but it is for producing an artificial valve 41 having a structure in which one end is folded back.

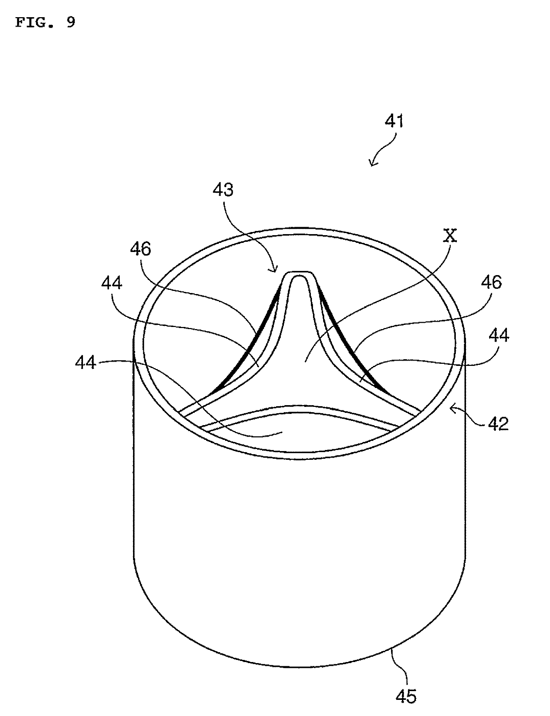

As illustrated in Figure 9, the artificial valve 41 is used as the aortic valve 18 or the like, for example, includes an outer pipe portion 42 made of a connective tissue 50 and a cylindrical valve body 43 provided on an inner side of the outer pipe portion 42, and a plurality of cusps 44 for opening/closing a channel X on the inner side of the valve body 43 are constituted.

The valve body 43 is formed having a shape in which one end of the outer pipe portion 42 is folded back to the inner side at a folding-back portion 45, and the valve body 43 is fixed to an inner surface of the outer pipe portion 42 on a wave-shaped fixing portion 46 so that this valve body 43 constitutes the plurality of cusps 44. The fixing portion 46 is formed by fixing the valve body 43 to the outer pipe portion 42 along a wave-shaped fixing line with a plurality of spots in the tip end portion of the valve body 43 as apexes, and this fixing portion 46 constitutes a support portion of the cusps 44. The fixing method of the valve body 43 to the outer pipe portion 42 is not particularly limited, and sewing, adhesion, fixing by a stapler and the like can be exemplified.

The cusps 44 are constituted one by one for each wave of the fixing portion 46, and the plurality of the cusps 44 are provided in parallel in the circumferential direction of the artificial valve 41. If there is a blood flow flowing from the tip end side toward the base end side (from an upper part to a lower part in Figure 9), in the cusps 44, a tip end portion of each of the cusps 44 receives a pressure and is deflected inward, and the plurality of the cusps 44 are brought into contact with each other, whereby the channel X inside the valve body 43 is closed. On the other hand, if there is a blood flow or the like flowing from the base end side toward the tip end side, the tip end portion of each of the cusps 44 receives the pressure, the cusps 44 deflected inward are returned, and the plurality of cusps 44 are separated away from each other, whereby the channel X inside the valve body 43 is opened.

As described above, it is so constituted that the cusps 44 reciprocate in a radial inward/outward direction with reversal of the direction of the blood flow or the like, whereby the channel X inside the valve body 43 is opened/closed, and the valve body 43 functions as the aortic valve having three cusps, for example, and opening/closing an aorta in a direction of the blood flow.

Subsequently, a base material 47 used when the artificial valve 41 described above is produced will be described.

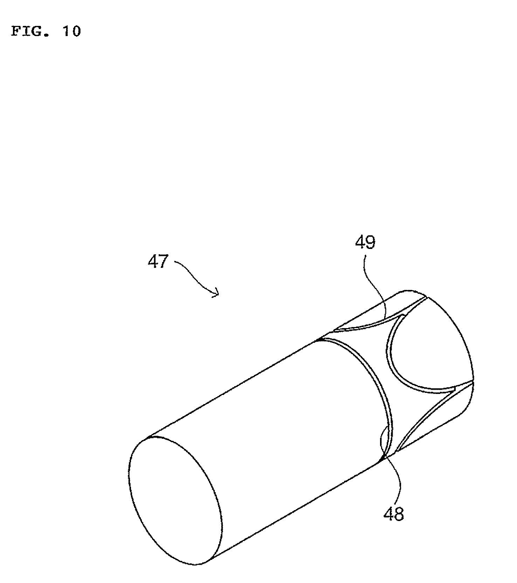

As illustrated in Figure 10, the base material 47 is for forming a tubular connective tissue structure 51 which can be machined to the artificial valve 41 on the surface thereof by being placed under the environment in which the biological tissue material is present and has a columnar shape forming the tubular connective tissue structure 51 on the surface.

This base material 47 has its length in the center axis direction set larger than the total sum of a length of the outer pipe portion 42 of the artificial valve 41 in the center axis direction and a length of the valve body 43 in the center axis direction so that the artificial valve 41 having the cylindrical valve body 43 can be formed inside the outer pipe portion 42 by folding back one end of the tubular connective tissue structure 11 inward.

In the vicinity of the center of the base material 47 in the center axis direction, a folding-back mark forming groove 48 as a folding-back mark forming portion continuing in the circumferential direction is formed, and it forms a folding-back mark 48a when the connective tissue structure 51 is folded. Moreover, on one end side of the base material 47, a wave-shaped fixing mark forming groove 49 as a fixing mark forming portion is formed and it forms a fixing mark 49a when the connective tissue structure 51 is fixed.

Subsequently, a method for producing the artificial valve 41 by using the base material 47 as above will be described.

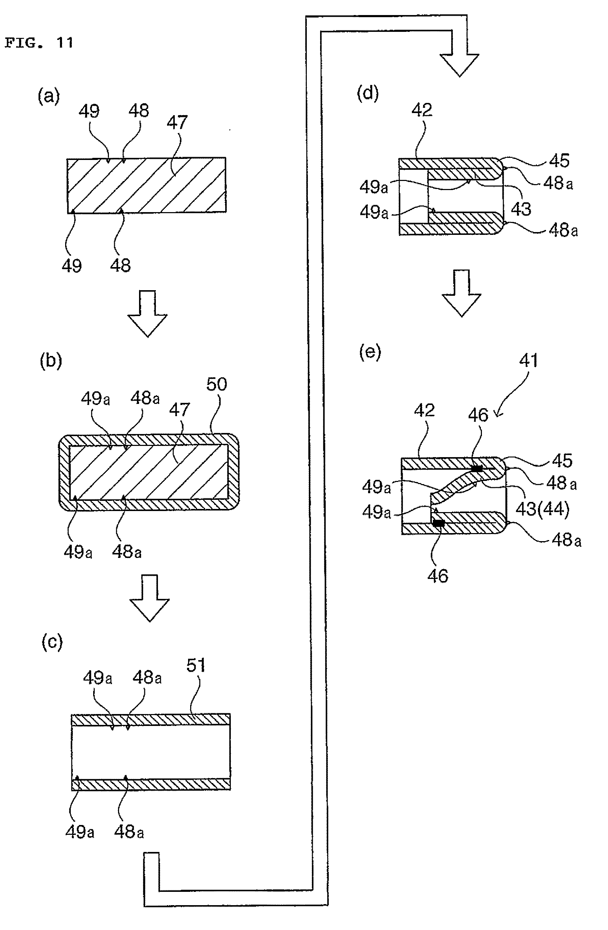

As illustrated in Figure 11, this production method includes an "installing process" of placing the columnar base material 47 under the environment in which the biological tissue material is present, a "forming process" of forming the membranous connective tissue 50 around the base material 47, a "taking-out process" of taking out the base material 47 covered with the connective tissue 50 from the environment, a "separating process" of separating and taking out the connective tissue 50 from the base material 47 as the tubular connective tissue structure 51, a "folding-back process" of forming the outer pipe portion 42 and the valve body 43 by folding back the one end of the tubular connective tissue structure 51, and a "fixing process" of fixing the valve body 43 to the outer pipe portion 42 along the fixing mark 49a so that the valve body 43 constitutes the plurality of cusps 44.

### <Installing process>

The base material 47 is placed under the environment in which the biological tissue material is present (Figure 11(a)).

### <Forming process>

After the installing process, as predetermined time elapses, the membranous connective tissue 50 is formed around the base material 47, the connective tissue 50 invades into the folding-back mark forming groove 48 and the fixing mark forming groove 49, and the folding-back mark 48a and the fixing mark 49a are formed (Figure 11(b)).

### <Taking-out process>

After the connective tissue 50 is sufficiently formed after the forming process of the predetermined time has elapsed, the taking-out process for taking out the base material 47 from the environment in which the biological tissue material is present is performed. The base material 47 taken out from the environment in which the biological tissue material is present is covered with a membrane made of the connective tissue 50 on the whole surface including its both end surfaces.

### <Separating process>

After the connective tissue 50 on the surface of the both end portions of the base material 47 is removed, the remaining connective tissue 50 covering the circumferential surface of the base material 47 is separated and taken out from the surface of the base material 47 as the tubular connective tissue structure 51 having the folding-back mark 48a and the fixing mark 49a (Figure 11(c)).

### <Folding-back process>

By using the position of the folding-back mark 48a as a folding-back portion 45, the one end of the tubular connective tissue structure 51 is folded back inward, and by making an outer side portion as the outer pipe portion 42 and a portion folded back inward as the valve body 43, the cylindrical valve body 43 is formed inside the outer pipe portion 42 (Figure (11d)).

### <Fixing process>

Along the wave-shaped fixing mark 49a, the valve body 43 folded back inside of the outer pipe portion 42 is fixed to the inner surface of the outer pipe portion 42 so as to constitute the wave-shaped fixing portion 46. As a result, the valve body 43 constitutes the plurality of cusps 44 having one wave portion of the fixing portion 46 as a support portion, and the artificial valve 41 having the plurality of cusps 44 inside the outer pipe portion 42 is obtained (Figure 11(e)).

The present invention is not limited to the aforementioned embodiments but is capable of changes as appropriate within a range of the present invention. For example, the artificial valve 1 in the first to third embodiments is not limited to the one used as an artificial mitral valve but can be also used as other artificial valves such as a tricuspid partitioning the right ventricle from the right atrium of the heart, for example, by setting the number of tendinous chords 7 and their fixing positions as appropriate.

Moreover, the base material for forming a connective tissue structure and the producing method according to the present invention can produce, not limited to the artificial valve 1, anything such as an artificial blood vessel, an artificial cornea and the like as long as it is a membranous connective tissue structure. Moreover, not only as the thickness-increasing groove for increasing the thickness and reinforcing the connective tissue structure on the base material surface but with the purpose of only making it easy to grasp the shape of the connective tissue structure, for example, a projection forming groove for forming a projection may be formed in the connective tissue structure.

### Reference Signs List

1 artificial valve (first embodiment)
2 heart
3 left ventricle
4 left atrium
5 valve body
6 fixing portion
7 tendinous chord
8 center hole
13 tendinous-chord tip end portion
14 anterior cusp
15 posterior cusp
16 right ventricle
17 aorta
18 aortic valve
19 base material
20 connective tissue structure
21 valve-body forming portion
22 tendinous-chord forming portion
23 notch
24 flange
25 groove portion
26 circular projection
27 knob
28 cutting-line forming groove
29 thickness-increasing groove
30 connective tissue
31 cutting line
32 base material (second embodiment)
33 valve-body forming portion
34 tendinous-chord forming portion
35 cutting-line forming groove
36 thickness-increasing groove
38 base material (third embodiment)
39a, 39b, 39c, 39d, 39e cutting-line forming groove for valve body
40 cutting-line forming groove
40a, 40b, 40c, 40d, 40e branch groove
41 artificial valve (fourth embodiment)
42 outer pipe portion
43 valve body
44 cusp
45 folding-back portion
46 fixing portion
47 base material
48 folding-back mark forming groove
48a folding-back mark
49 fixing mark forming groove
49a fixing mark
50 connective tissue
51 connective tissue structure
X channel

## Claims

1. A base material for forming a membranous connective tissue structure on a surface of the base material by being placed under an environment in which a biological tissue material is present, wherein
a working mark forming portion for forming a mark when the connective tissue structure is worked is formed on the base material surface.

2. The base material for forming a connective tissue structure according to claim 1, wherein
a cutting-line forming groove for forming a cutting line when the connective tissue structure is to be cut is formed as the working mark forming portion.

3. The base material for forming a connective tissue structure according to claim 1 or 2, wherein
a folding-back mark forming portion for forming a folding-back mark when the connective tissue structure is folded is formed as the working mark forming portion.

4. The base material for forming a connective tissue structure according to claim 1, 2 or 3, wherein
a fixing mark forming portion for forming a fixing mark when the connective tissue structure is fixed is formed as the working mark forming portion.

5. The base material for forming a connective tissue structure according to any one of claims 1 to 4, wherein
a projection forming groove forming a projection on the connective tissue structure is formed on the base material surface separately from the working mark forming portion.

6. The base material for forming a connective tissue structure according to claim 2, wherein
a projection forming groove for forming a projection in the connective tissue structure is formed on the base material surface separately from the cutting-line forming groove; and
the projection forming groove is provided in parallel with the cutting-line forming groove in a groove width direction.

7. The base material for forming a connective tissue structure according to claim 5 or 6, wherein
the projection forming groove is formed as a thickness-increasing groove which increases thickness and reinforces the connective tissue structure.

8. A method for producing a membranous connective tissue structure made of a connective tissue, comprising:
an installing step of placing a base material having a cutting-line forming groove on a base material surface under an environment in which a biological tissue material is present;
a forming step of forming a cutting line by causing the connective tissue to invade into the cutting-line forming groove while the connective tissue is formed around the base material;
a taking-out step of taking out the base material covered with the connective tissue from the environment;
a separating step of separating and taking out the connective tissue covering the base material surface as the membranous connective tissue structure; and
a cutting step of cutting the connective tissue structure along the cutting line.

9. A method for producing a membranous connective tissue structure made of a connective tissue, comprising:
an installing step of placing a base material having a working mark forming portion on a base material surface under an environment in which a biological tissue material is present;
a forming step of forming a mark by causing the connective tissue to invade into the working mark forming portion while the connective tissue is formed around the base material;
a taking-out step of taking out the base material covered with the connective tissue from the environment;
a separating step of separating and taking out the connective tissue covering the base material surface as the membranous connective tissue structure; and
a working process of working the connective tissue structure at the position of the mark.
